Europäisches Patentamt

European Patent Office

Office européen des brevets

(19)

(11) Publication number: **0 199 855**

**A1**

(12) # EUROPEAN PATENT APPLICATION

(21) Application number: **85200692.3**

(22) Date of filing: **02.05.85**

(51) Int. Cl.⁴: **A 61 K 31/635**
**A 61 K 9/20**

(43) Date of publication of application:
**05.11.86 Bulletin 86/45**

(84) Designated Contracting States:
**NL**

(71) Applicant: **Gist - Brocades N.V.**
**Wateringseweg 1 P.O. Box 1**
**NL-2600 MA Delft(NL)**

(72) Inventor: **Tan, Hong Sheng**
**Buizerdstraat 94**
**NL-2665 TG Bleiswijk(NL)**

(72) Inventor: **Wegman, Bernardus Bartholomeus Maria**
**Norholm 58**
**NL-2133 HL Hoofddorp(NL)**

(74) Representative: **Schmieman, Johannes Hendrik et al,**
**Gist-Brocades N.V. Patents & Trademarks Department**
**Martinus Nijhofflaan 2 P.O. Box 1**
**NL-2600 MA Delft(NL)**

(54) Tablets comprising tromethoprim and a sulfonamide.

㊲ The invention relates to tablets containing trimethoprim or a salt thereof and a sulfonamide or a salt thereof and an ion exchanger of the artificial resin type. The total weight of the active ingredients is from 80 to 98% of the tablet weight.

EP 0 199 855 A1

This invention relates to tablets containing an admixture of 2,4-diamino-5-(3,4,5-trimethoxybenzyl)pyrimidine (trimethoprim) and a sulfonamide. Pharmaceutical compositions comprising said ingredients are well known for the treatment of microbially-infected animals and human beings. They usually contain trimethoprim and a sulfonamide in a ratio of 1:5 and the dosages of the mixture commonly given to adult patients are from 100 to 1000 mg.

When conventional pharmaceutical techniques are employed, the amount of active ingredients in a tablet should not exceed 80% (w/w). A larger percentage will give tablets with poor physical properties, such as a high disintegration or dissolution time, a high friability value or a low hardness value. Conventional tablets containing high doses of trimethoprim and a sulfonamide consequently have a large size, which make them difficult to swallow for a patient.

Attempts have been made to produce tablets with more than 80% of the said ingredients which still show satisfactory physical characteristics. Such a tablet is disclosed in GB 1499672. According to this patent tablets containing trimethoprim and a sulphonamide (e.g. sulphamethoxazole) are prepared which contain 80 to 98% (w/w) of active ingredients. In addition to the active ingredients the tablets comprise a disintegrating agent with a swelling capacity greater than 5 ml/g and a granulating agent, and the particle size, defined in terms of weight median diameter, of the active ingredients should be less than 40 m.

It has now been found that tablets comprising a large amount of trimethoprim and a sulfonamide and having satisfactory physical properties can be obtained by incorporating in them as a disintegrating agent an ion exchanger of the artificial resin type. The use of ion exchangers in the preparation of solid pharmaceutical preparations is already known, e.g. from GB 791,281, but this patent specification does not disclose a method for the preparation of compositions with a high percentage of active ingredients. The compositions described contain a relative large amount of other excipients, particularly lactose, which by its hydrophilic character promotes the penetration of liquid in the tablet.

According to the invention tablets comprising a total amount of trimethoprim and a sulfonamide from 80 to 98% (w/w) in a ratio of 1:20 to 20:1 are obtained by incorporating in them an ion exchanger of the artificial resin type. The tablets of the invention do not need a large amount of lactose or other hydrophilic agents such as maize starch or microcrystalline cellulose.

It will be understood that the ion exchanger should be pharmaceutically acceptable. This implies that the ion exchanger should not have a biological effect of its own in the doses used and that it should have a high degree of purity and a small particle size. Pharmaceutical grade ion exchangers, meeting these requirements, are available, inter alia under the trade name Amberlite.

In view of the slight solubility of trimethoprim it would be expected that tablets with an acceptable disintegration time could only be obtained by using a swelling agent with a high swelling capacity, as described in GB 1499672. It has surprisingly been found that such a swelling agent is not necessary in the tablets of the invention. Moreover, the ion exchangers used in the tablets have the advantage that they do not give a slimy, sticky mass in contact with a liquid.

Preferred ion exchangers are copolymers of divinylbenzene with styrene or methacrylic acid and phenol-polyamines, particularly the divinylbenzene copolymers. Most preferred are weakly acid cation exchangers consisting of methacrylic acid-divinylbenzene copolymer with free carboxyl groups, such as Amberlite IRP-88. The ion exchanger is preferably used in an amount from 1 to 8% (w/w) of the total tablet. More preferably 2-6% is used. The ratio of trimethoprim and sulfonamide is preferably 1:5. A preferred sulfonamide is sulfamethoxazole (3-(4-aminobenzenesulphonamido)-5-methylisoxazole.

The tablets may further contain excipients commonly used in pharmacy, such as filling, binding, swelling, lubricating, wetting, granulating, flavoring and colouring agents. Examples of such additives are polyvinylpyrrolidone, magnesium stearate, highly purified silicon dioxide, sodium dioctylsulfosuccinate, cellulose derivatives (e.g. sodium carboxymethylcellulose) and gelatin.

For the purpose of the invention trimethoprim and the sulfonamide may be used as such or in the form of pharmaceutically acceptable salts, i.e. salts which are not harmful for the human or animal organism in the doses to be administered. Examples of such salts are hydrohalides (e.g. the hydrochloric or the hydrobromide), the citrate, lactate, glutaminate and methylsulfonate of trimethoprim and the salts of sulfonamides with bases such as sodium hydroxide, ammonia, and amines (e.g. alkanolamines such as ethanolamine).

According to a feature of the invention the tablets are prepared by making a mixture comprising trimethoprim and a sulfonamide, granulating the mixture with a suitable liquid, mixing the granulate with the other ingredients and compressing tablets from the mixture obtained. The granulating liquid is preferably a protic solvent, such as ethanol or water or mixtures thereof, which may contain one or more additives, such as binding and wetting agents, for instance polyvinylpyrrolidone, sodium dioctylsulfosuccinate, gelatin or sorbitol. The preferred solvent is ethanol. The ion exchanger may be mixed with the active agents to be incorporated in the granulate, but it may also be added entirely or partly after granulation.

The tablets of the invention show satisfactory hardness and friability and they comply with the usual standards with respect to disintegration time.

- 4 -

The invention also includes dispersable tablets and their preparation. Such tablets may be obtained by incorporating a relatively large amount of the disintegrating agent, preferably 4-6%. Suitable disintegrating agents for dispersable tablets are Amberlite resins of the types IRP88, IRP 67M and IRP 69M.

The preparation of tablets according to the invention is illustrated by the following Examples.

The tablets were compressed on a Hoko excenter press. The hardness was measured with a Schleuniger 2E apparatus and the disintegration time was determined with an Erweka disintegration apparatus according to BP 80. In Examples 4, 5 and 6 the disintegration was measured in water of 20°C, in the other Examples in water of 37°C.

## Example 1

In a Turbula mixer 1200 g of sulfamethoxazole, 240 g of micronised trimethoprim and 28.8 of Amberlite IRP.88 were mixed for 15 minutes. The mixture was granulated in a Hobart mixer with a solution of 28.8 g of PVP K30 in 350 ml of a water-ethanol mixture (1:1 v/v); after 10 minutes another 150 ml of the water-ethanol mixture were added. The moist mixture was ground in an Apex mill, spread out on trays and dried overnight at 40-45°C. The granulate was sieved through a Frewitt apparatus with a 1,25 mm screen. The sieved granulate was mixed for two minutes in a Turbula mixer with 5 g of magnesium stearate and 5 g of Aerosil 200 V (highly purified silicon dioxide) per kg of granulate. The mixture obtained was pressed into tablets with a diameter of 11 mm, a hardness of 120 N and a weight of 520 mg. Friability: 0.3%; disintegration time : 45 sec.

Further, oblong tablets were compressed with a hardness of 170 N and a weight of 1020 mg. Friability: 0.4%; disintegration time: 45 sec.

## Example 2

A mixture of 400 g of sulfamethoxazole and 80 g of micronised trimethoprim was granulated in an Erweka kneader with a solution of 9.6 g of PVP K30 in 160 ml of ethanol/water (1:1 v/v). The granulate obtained was dried at 50°C and then sieved through a Frewitt apparatus with a 1.0 mm screen. The granulate was mixed with 9.6 g of Amberlite IRP88 for 10 minutes on a rolling road. After addition of 2.5 of magnesium stearate and 2.5 g of Aerosil 200 V, tablets were pressed with a diameter of 11 mm and a weight of 480 mg. Hardness: 100 N; disintegration time:  30 sec.

## Example 3

a. In a Topo-granulator (mini-Topo) a mixture of 600 g of micronised sulfamethoxazole, 120 g of micronised trimethoprim and 14.4 g of Amberlite IRP 88 was granulatd with a solution of 14.4 g of PVP K30 in 275 ml of ethanol. The granulate was then dried in the mini-Topo and sieved through a Frewitt apparatus with a 1.0 mm screen.
A part of the granulate was mixed with 0.5% of Aerosil 200 V and 0.75% of magnesium stearate and then compressed into tablets with a diameter of 11 mm and a weight of 522 mg. Hardness:  110 N;  disintegration time:  30 sec.

b. Another part of the granulate was sieved through a Frewitt apparatus with a 0.63 mm screen, 49 g of the sieved fraction were mixed with 1.5 g of Amberlite IRP88, 0.75% of magnesium stearate and 0.50 of Aerosil 200 V and the mixture was compressed into tablets with a diameter of 13 mm and a weight of 860 mg. Hardness: 70 N; disintegration time: 20 sec.

## Example 4

80 g of micronised sulfamethoxazole, 16 g of micronised trimethoprim and 5.0 g of Amberlite IRP 88 were mixed in a Hobart mixer. After 5 minutes a granulating liquid consisting of 2.0 g of PVP K30, 0.04 g of Aerosol OT (sodium dioctylsulfosuccinate) and 40 ml of ethanol were added under stirring. A granulate was obtained, which was dried on a fluid bed drier and pressed through a 0.80 mm screen. The granules were mixed with 0.5 g of Aerosil 200 V and 0.75 g of magnesium stearate and tablets were compressed with a diameter of 13 mm and a weight of 730 mg. Hardness: 140 N; disintegration time: less than 1 minute.

## Example 5

The procedure of Example 4 was followed, but instead of 2.0 g of PVP K30, 1.0 g was used. The tablets had a hardness of 150 N, a weight of 660 mg and a disintegration time of less than 1 minute.

## Example 6

80.0 g of micronised sulfamethoxazole, 16.0 g of micronised trimethoprim, 5.0 g of Amberlite IRP 88 and 5.0 g of Elcema G 250 (a cellulose derivate) were mixed in a Hobart mixer and granulated with a solution of 1.0 g of PVP K30 in 50 ml of ethanol. The granulate was dried on a fluidized bed and pressed through a 0.80 mm screen. The granulate was mixed for two minutes with 0.75 g of magnesium stearate and 0.50 g of Aerosil 200 V and tablets were compressed with a diameter of 13 mm and a weight of 678 mg. Hardness: 140 N; disintegration time: less than 1 minute.

## Example 7

In a Hobart mixer, 60 mg of micronised sulfamethoxazole, 12 g of micronised trimethoprim and 1.4 g of Amberlite IRP 88 were mixed for 5 minutes and the mixture was granulated with a solution of 1.55 g of gelatin in 30 ml of ethanol/water (1:1 v/v). The granulate was dried at 60°C and pressed through a 1.0 mm screen. The granules were mixed for two minutes wih 0.75% of magnesium stearate and 0.5% of Aerosil 200 V in a Turbula mixer and convex tablets were pressed with a diameter of 11 mm and a weight of 523 mg. Hardness: 130 N; disintegration time: 25 sec.

## Example 8

The procedure of Example 7 was followed but 1.5 g of sorbitol was used instead of gelatin. The tablets had a weight of 550 mg, a hardness of 170 N and a disintegration time of 60 sec.

## Example 9

60 g of micronised sulfmethoxazole and 12 g of micronised trimethoprim were mixed for 5 minutes in a Hobart mixer. The mixture was granulated with a solution of 1.5 g of gelatin in 30 ml of ethanol/water (1:1 v/v). The granulate was dried at 60°C and pressed through a 1.0 mm screen. The granules were mixed for 10 minutes with 1.4 g of Amberlite IRP 88 and subsequently for 2 minutes with 0.75% magnesium stearate and 0.5% Aerosil 200 V. Tablets were compressed with a diameter of 11 mm and a weight of 525 mg. Hardness: 140 N disintegration time: 35 sec.

## Example 10

The procedure of Example 9 was followed, but 1.5 g of sorbitol was used instead of gelatin. The tablets with a weight of 540 mg and a hardness of 170 N disintegrated in 90 seconds.

## Example 11

In a Hobart planetary mixer 60 g of micronised sulfmethoxazole, 12 g of trimethoprim and 1.4 g of Amberlite IRP 88 were stirred for 5 minutes and the mixture was granulated with 32 ml of ethanol/water (1:1 v/v). The granulate was dried at 60°C and pressed through a 1.0 mm screen. The granules were mixed for two minutes with 0.75% of magnesium stearate and 0.5% of Aerosil 200 V in a Turbula mixer and convex tablets were pressed with a diameter of 11 mm and a weight of 540 mg. Hardness: 160 N; disintegration time 15 sec.

## Example 12

In a Hobart planetary mixer 60 g of micronised sulfmethoxazole and 12 g of micronised trimethoprim were mixed for five minutes. The mixture was granulated with 30 ml of ethanol/water (1:1 v/v). The granulate was dried at 60°C and it was then comminuted in a Frewitt apparatus with a 1.0 mm screen. The granules were mixed for 10 minutes on a rolling road with 1.4 g of Amberlite IRP 88 and then 0.75% magnesium stearate and 0.5% Aerosil 200 V were added, after which mixing was continued for two minutes. The mixture was compressed into convex tablets with a diameter of 11 mm and a weight of 535 mg. Hardness: 150 N; disintegration time 15 sec.

## Example 13

A mixture of 80 g of micronised sulfmethoxazole, 16 g of micronised trimethoprim and 4.8 g of Amberlite IRP 88 was granulated with a solution of 50 mg of Aerosol OT in 45 ml of ethanol. The moist mass was pressed through a 2.0 mm screen and it was then dried in a vacuum stove at 60°C. The dried granulate was sieved through a 0.8 mm screen and mixed with 0.75% magnesium stearate and 0.5% Aerosil 200 V. Tablets were pressed with a diameter of 15 mm and a weight of 1000 mg. Hardness: 160 N; disintegration time: 15 sec.

## Example 14

I Preparation of granulate

In an Erweka kneader 400 g of micronised sulfamethoxazole and 80 g of micronised trimethoprim were mixed and subsequently granulated with a solution of 9.6 g of polyvinylpyrrolidone K30 and 0.6 g of Aerosol OT in 150 ml of alcohol. The granulate was dried at 60°C and sieved in a Frewitt apparatus with a 1.0 mm screen.

II Preparation of tablets

a. 40 g of the granulate was mixed for 10 minutes with 1.6 g of Amberlite IRP 64M (weakly acid ion exchanger of the $H^+$ form). Then 0.200 g of Aerosil 200 V and 0.300 g of magnesium stearate were added and mixing was continued for 2 minutes.
Tablets were pressed with a diameter of 13 mm and a weight of 710 mg. Hardness: 160 N;  disintegration time: 6 minutes.

b. The procedure described under a was followed, but Amberlite IRP 67M (strongly basic ion exchanger of the $Cl^-$ form) was used instead of Amberlite IRP 64M. The tablets had a weight of 705 mg, a hardness of 130 N and a disintegration time of less then one minute.

c. The procedure described under a was followed, but Amberlite IRP 69M (strongly acid ion exchanger in the Na+ form) was used instead of Amberlite IRP 64 M. The tablets had a weight of 715 mg, a hardness of 130 N and a disintegration time of less than one minute.

d. The procedure described under a was followed, but Amberlite IRP 88 was used instead of Amberlite IRP 64 M. The tablets had a weight of 675 mg, a hardness of 200 N and a disintegration time of less than one minute.

## Example 15

20 g of sulfadiazine and 4 g of trimethoprim were mixed and the mixture was granulated with 12 ml of a solution of 1% (w/v) polyvinylpyrrolidone in water/ethanol (1:1). The granulate was dried at 60°C and it was then pressed through a 1.0 mm screen. The granules were successively mixed with 2% Amberlite IRP-88, 0.5% Aerosil 200V and 0.5% magnesium stearate. The mixture was compressed into convex tablets with a diameter of 11 mm and a weight of 530 mg. The hardness was 120 N and the disintegration time less than 60 seconds.

## Example 16

A mixture of 30 g of sulfamethoxazole and 2 g of trimethoprim was granulated with a solution of 0.64 g of polyvinylpyrrolidone in 12 ml of ethanol/water (1:1). The granulate was dried at 60°C and it was then pressed through a 1.0 mm screen and further mixed with successively 2% Amberlite IRP-88, 0.5% Aerosil 200V and 0.5% magnesium stearate.

Convex tablets were compressed with a diameter of 11 mm and a weight of 600 mg. The hardness was 200 N and the disintegration time less than 30 seconds.

## Example 17

The procedure of Example 16 was followed, using 30 g of trimethoprim instead of 2 g and 2 g of sulfamethoxazole instead of 30 g. The convex tablets had a diameter of 11 mm, a weight of 538 g, a hardness of 60 N and a disintegration time less than 15 seconds.

## CLAIMS

1. Tablets containing trimethoprim or a salt thereof and a sulfonamide or a salt thereof in a ratio between 1:20 and 20:1, the total weight of said ingredients being from 80 to 98% of the total weight of the tablet, characterized in that they comprise an ion exchanger of the artificial resin type.

2. Tablets according to claim 1, characterized in that the ion exchanger is a copolymer of divinylbenzene with styrene or methacrylic acid or a phenol-polyamine.

3. Tablets according to claim 2, characterized in that the ion exchanger is a copolymer of divinylbenzene with styrene or methacrylic acid.

4. Tablets according to claim 3, characterized in that it contains a weakly acid cation exchanger consisting of methacrylic acid-divinylbenzene copolymer with free carboxyl groups.

5. Tablets according to any of the claims 1-4, characterized in that the amount of ion exchanger is between 1 and 8% (w/w) of the total tablet.

6. Tablets according to claim 5, characterized in that the amount of ion exchanger is between 2 and 6% (w/w) of the total tablet.

7. Tablets according to any of the foregoing claims, characterized in that the weight ratio of trimethoprim to sulfonamide is 1:5.

8. Tablets according to any of the foregoing claims, characterized in that the sulfonamide is sulfamethoxazole.

9. Tablets according to any of the foregoing claims characterized in that they are dispersible tablets containing 4 to 6% (w/w) of a disintegrating agent.

10. Process for the preparation of tablets as defined in claim 1, characterized in that a mixture is made, comprising trimethoprim or a salt thereof and a sulfonamide or a salt thereof, the mixture is granulated with a suitable liquid, the granulate is mixed with one or more other ingredients and tablets are compressed from the mixture and an ion exchanger of the artificial resin type is added during the preparation of the granulate and/or added to the granulate.

11. Process according to claim 10, characterized in that the liquid used in the granulation is a protic solvent, optionally containing additives such as binding and wetting agents.

12. Process according to claim 11, characterized in that the protic solvent is ethanol.

**European Patent Office**

**EUROPEAN SEARCH REPORT**

EP 85 20 0692

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | GB-A-2 067 900 (DDSA PHARMACEUTICALS) * Whole document * | 1-12 | A 61 K 31/635 A 61 K 9/20 |
| Y | CHEMICAL ABSTRACTS, vol. 97, no. 4, July 1982, page 305, no. 28529f, Columbus, Ohio, US; C. BOYMOND et al.: "Effect of different disintegration agents on the dissolution rate for phenacetin tablets", & PHARM. ACTA. HELV. 1982, 57(5-6), 131-5 | 1-12 | |

|  |  |  | TECHNICAL FIELDS SEARCHED (Int. Cl 4) |
|---|---|---|---|
|  |  |  | A 61 K |

The present search report has been drawn up for all claims

| Place of search THE HAGUE | Date of completion of the search 10-01-1986 | Examiner BENZ K.F. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82